# EUROPEAN PATENT APPLICATION

(11) **EP 4 245 847 A1**
(43) Date of publication of application: **20.09.2023**
(21) Application number: 21892057.7
(22) Date of filing: 10.05.2021
(51) Int. Cl.: C12N 5/0783, C12N 5/00, C12M 1/12

(54) **MASS PROLIFERATION CULTURE METHOD OF NK CELLS**

(30) Priority: 11.11.2020 KR 20200150118
(71) Applicant: Hanbio Co., Ltd., Seoul 06232 (KR); Kang, Da Witt, Seoul 06356 (KR)
(72) Inventor: KANG, Hyun Chul, Bucheon-si Gyeonggi-do 14426 (KR); KANG, Da Witt, Seoul 06356 (KR)
(74) Representative: Ipside
(86) International application number: PCT/KR2021/005785
(87) International publication number: WO 2022/102887

(57) **Abstract**

The present invention relates to a method for culturing NK cells, and more particularly, to a method for culturing NK cells method comprising the steps of: 1) separating a pellet of immune cells of peripheral blood mononuclear cells (PBMCs) from blood; 2) culturing the PBMCs in an RPMI culture medium in an incubator coated with an anti-CD16 antibody; 3) subculturing the PBMCs; and 4) activating the NK cells by treating the PBMCs with a cytokine selected from a cytokine group consisting of IL-2, IL-15, and IL-2+IL-15. The present invention is an optimal combination that can effectively activate cells when NK cells are treated with IL-2 and IL-15 among various cytokines, and NK cells can be mass-cultured when treated with IL-2+IL-15. Also, when the NK cells are used, apoptosis or killing ability of cancer cells can be promoted, and thus, the NK cells can be used as an effective immune cell therapeutic agent for the prevention or treatment of cancer.

## Description

### TECHNICAL FIELD

The present invention relates to a mass proliferation culture method of natural killer cell (NK cell) applied to immunotherapy, more particularly, to a mass proliferation culture method of NK cell capable of culturing lymphocytes derived from human peripheral blood to efficiently proliferate and activate NK cells that are highly effective in treating malignant tumors and at the same time, to significantly increase the proportion occupied by NK cells.

### BACKGROUND ART

Immunotherapy is a method for extracting immune cells that are the most important to cancer treatment, such as natural killer cells (NK cells), dendritic cells (DC), B cells, and T cells, from a patient's blood, culturing the cells into immune cells that strongly acts against cancer by using various types of stimulants, and injecting the same back to the patient. There are fewer side effects than conventional chemotherapy, since a patient's own blood is used, and the administration method is simple. Therefore, immunotherapy has been actively studied recently.

Among the immune cells that are activated in immunotherapy, NK cells, in particular, are large granular lymphocytes (LGL), a characteristic type of lymphocyte, have an excellent capability to kill infected viruses and tumor cells without killing most normal cells. The antitumor action is accomplished by necrosis or apoptosis, or by the simultaneous occurrence of these two action mechanisms. NK cells respond to cytokines such as IL-2, IL-12, and interferon, thereby increasing cytotoxicity and secretory and proliferative functions. The phenotype of NK cells in humans is CD16 (FcγRIII) and CD56, wherein CD16 and CD56, which lack TRC (T-cell receptor complex) on the cell surface, are used as markers for NK cells.

These NK cells are known to play an important role in the early biophylaxis mechanism and tumor immunity of the human body.

In other words, NK cells can kill specific autologous cells, allogeneic cells, and even heterogeneous cancer cells without the process of acquiring immunity according to the expression of Major Histocompatibility Complex (MHC), and are capable of more effectively killing target cells that express little or no Class 1 MHC. Therefore, NK cells can effectively kill most cancer cells that do not express MHC, and can kill some virus-infected cells and bacteria such as *Salmonella typhi.*

These NK cells are important cells that are responsible for innate immunity, and unlike T cells, they become mature in the liver and bone marrow. In particular, they have a function to autonomously identify and kill abnormal cells, such as virus-infected cells or tumor cells. In the last decade, tumor immunotherapy using patient's own immune system has been steadily developed, and 'cell therapy products' using the same have also been commercialized. Cell therapy is defined as a pharmaceutical product used for treatment, diagnosis, and prevention by a series of actions that change the biological characteristics of cells through an *in vitro* method for proliferating and selecting autologous, allogeneic, or xenogeneic cells (Article 2 of the Food and Drug Administration Notification No. 2003-26).

Since NK cell is a cell therapy for the treatment of intractable diseases such as cancer and is capable of resolving immunorejection and providing patient-tailored cell therapy, there is a need for a mass proliferation method of NK cell and a method for culturing NK cells with enhanced activity.

However, NK cells, which have an excellent effect on killing cancer cells, account for only 5 to 15% of peripheral blood lymphocytes even in a normal person, and especially in cancer patients, the proportion is lowered to less than 1%. Therefore, NK cells have limitations in effectively attacking cancer cells in the absence of a separate proliferation process through immunotherapy.

The differentiation, proliferation, and survival of NK cells are greatly influenced by cytokines in terms of their function, and several previous studies have shown that cytokines such as IL-2, IL-12, IL-15, and IL-18 increase cell toxicity and activation. Currently, cytokines such as IL-2 and antibodies such as CD3 are used to activate and proliferate immune cells, particularly NK cells. In the current technology, the CD3 antibody plays a critical role in the proliferation of immune cells, but the problem is that activating immune cells by using this antibody is very difficult.

In particular, when strong stimulation of the CD3 antibody is applied from the beginning, immature progenitor cells mature into T cells. Therefore, the current method that is generally performed employes a method of slightly stimulating the CD3 antibody in the beginning, which is highly dependent upon the environmental requirements such as differences among individuals. Therefore, it is challenging to obtain activated NK cells that are proliferated in large quantities. Therefore, there is a need to develop a new culture medium composition and a new culture method that can stably amplify and proliferate NK cells in large quantities while eliminating the difficulty of the current method that the CD3 antibody should be immobilized in a flask and subject to a reaction for a certain period of time.

Therefore, while contemplating a mass proliferation culture method of NK cell using cytokines, the present inventors isolated and cultured lymphocytes, which are immune cells derived from human peripheral blood, and confirmed that when various cytokines, particularly IL-2 and IL-15, were treated together in an incubator coated with anti-CD16 antibody rather than anti-CD3 antibody, the NK cells were massively proliferated and activated, thereby completing the present invention.

### DETAILED DESCRIPTION OF THE INVENTION

### TECHNICAL PROBLEM

The purpose of the present invention is to provide a mass proliferation culture method of NK cell using cytokines.

### TECHNICAL SOLUTION

To achieve the purpose above, the present invention provides a culture method of NK cell, the method comprising: 1) isolating a pellet of peripheral blood mononuclear cell (PBMC) containing immune cells from blood by using ficoll and centrifugation; 2) culturing the isolated PBMC in an anti-CD16 antibody-coated incubator in a medium containing RPMI culture medium, plasma, IL-2, IL-15, anti-CD56, anti-NKp46, and anti-NKp30; 3) subculturing the cultured PBMC in RPMI medium, albumin, IL-2, IL-15, anti-CD56, anti-NKp46, and anti-NKp30; and 4) treating the subcultured PBMC in a medium composition based on RPMI to which anti-NKp30, anti-NKp46 and anti-CD56 antibodies are added with a cytokine selected from a cytokine group consisting of IL-2, IL-15, and IL-2 + IL-15 to activate NK cells.

Hereinafter, the present invention will be described in detail.

NK cells are large granular lymphocytes (LGL), a type of lymphocyte, and have an excellent capability to kill infected viruses and tumor cells without killing most normal cells. Thus, NK cells play an important role in the early stage of viral infection or tumorigenesis before active cytotoxic T lymphocytes are produced in a large quantity. For example, when an NK cell contacts a target cell, some molecules form pores on the target cell's membrane and lyse the cell, while other molecules enter into a target cell and increase fragmentation of nuclear DNA, causing necrosis, apoptosis or programmed cell death.

Therefore, NK cells can lyse specific virus-infected cells and cancer cells without stimulating them in advance. Unlike cytotoxic T lymphocytes, which recognize antigen-specifically through expression of TCR, NK cells lack antigen-specific receptors.

NK cells express a killer cell immunoglobulin-like receptor (KIR) that binds to MHC type I of normal cells. Killer cell inhibitory receptors, when combined with MHC class I, generate intracellular signals that induce the inhibition of specific transcription factors. As a result, the activation of NK cells and the degradation and destruction of target cells are inhibited. In virus-infected cells or cancer cells, the MHC class I molecules are greatly reduced on their surface. Therefore, when these cells encounter NK cells, because they do not effectively bind to killer cell inhibitory receptors, they are exposed to NK cell-mediated cytotoxicity and thus are lysed.

The NK cells may be derived from, for example, mammals, humans, monkeys, pigs, horses, cows, sheep, dogs, cats, mice or rabbits. The NK cells may be obtained from a normal person or a cancer patient. The NK cells may be isolated from blood or peripheral blood mononuclear cell (PBMC). A method of isolating blood, a method of isolating PBMC therefrom, and a method of isolating NK cells therefrom may be performed by known methods.

In the NK cell culture method of the present invention, the cytokine of Step 4) above is preferably treated with IL-2 + IL-15, and more preferably, IL-2 20 ng/ml and IL-15 50 ng/ml are treated.

In addition, in the NK cell culture method of the present invention, red blood cells are preferably removed by treating an immune cell pellet with an RBC lysis buffer when isolating immune cells in Step 1) above; and the culturing in Step 2) above is preferably carried out in a T25 flask coated with anti-CD16 antibody when the cell number is 3×10⁷ or less and in a T75 flask coated with anti-CD16 antibody when the cell number is 3×10⁷ or more by adding RPMI 8 ml, plasma 2 ml, IL-2 20 ng/ml, IL-15 50 ng/ml, anti-CD56 5 ng/ml, anti-NKp46 5 ng/ml, and anti-NKp30 5 ng/ml, suspending PBMC pellet in 10 ml RPMI and further adding the mixture, and culturing in an incubator at 37°C and 5% CO₂ for 3 to 4 days.

In addition, in the NK cell culture method of the present invention, the subculturing in Step 3) above is preferably carried out by adding the cell culture suspension of Step 2) above 20 ml, RPMI 72 ml, albumin 8 ml, IL-2 20 ng/ml, IL-15 50 ng/ml, anti-NKp30 5 ng/ml, anti-NKp46 5 ng/ml, and anti-CD56 5 ng/ml and culturing in an incubator at 37°C and 5% CO₂ for 3 to 5 days in primary subculture; and by adding primary subculture suspension 50 ml, RPMI 180 ml, albumin 20 ml, IL-2 20 ng/ml, IL-15 50 ng/ml, anti-NKp30 5 ng/ml, anti-NKp46 5 ng/ml, and anti-CD56 5 ng/ml and culturing in an incubator at 37°C and 5% CO₂ for 5 to 7 days in secondary subculture.

### ADVANTAGEOUS EFFECTS

As described above, the present invention is an optimal combination that can effectively activate cells when NK cells are treated with IL-2 and IL-15 among various cytokines, and when treated with IL-2 + IL-15, mass culture of NK cells is possible, and in particular, the culture is most effective when treated with 20 ng/ml of IL-2 and 50 ng/ml of IL-15. Since apoptosis or killing ability of cancer cells can be promoted when this is used, the present invention can be used as an effective immune cell therapy for preventing or treating cancer.

### DESCRIPTION OF THE DRAWINGS

FIG. 1 shows the results of flow cytometry according to the distribution (distribution of NK cells, NKT cells, and T cells) according to CD3 and CD56 antibodies for each of three types of immune cell PBMC samples isolated according to Example 1. The X-axis of the graph in FIG. 1 indicates CD3 (T lymphocyte marker) and the Y-axis indicates CD56 (NK lymphocyte marker).
FIG. 2 is a graph showing the results of measuring the amount of IFN-γ secretion according to the IL-2 and IL-15 cytokine treatment concentration in NK cells.
FIG. 3 is a graph showing the results of measuring the activity according to the amount of secretion of IFN-γ after setting the optimal concentrations of IL-2 and IL-15 cytokines according to FIG. 2 and treating the same at the set concentrations.
FIG. 4 is a graph showing the results of measuring the distribution of immune cells according to antibody and IL-2 and IL-15 cytokine treatment under different conditions.
FIG. 5 is a graph showing the total cell count and cell viability of immune cells according to antibody and IL-2 and IL-15 cytokine treatment under different conditions
FIG. 6 is a graph showing the number of cells measured after culturing NK cells for 14 days under set conditions.
FIG. 7 is a graph showing the results of FACS analysis test according to the NK cell mass culture composition under set conditions.

### MODE OF THE INVENTION

Hereinafter, the present invention will be described in more detail through Examples. These Examples are only for explaining the present invention in more detail, and it will be obvious to one of ordinary skill in the art that the scope of the present invention is not limited by these Examples according to the gist of the present invention.

### <Example 1> Isolation of immune cells from blood

To isolate immune cells from blood, ficoll (GE Healthcare), PBS (WELGENE), two filter tubes (Greiner bio-one), four 50 ml tubes (VWR), an EDTA tube (BD biosciences), and a hematocytometer (MARIENFELD) were first prepared.

Blood of 50 to 100 ml, collected in a sterilized EDTA tube, was transported to a preparation room, and the blood was divided into 50 ml tubes. Ficoll of 15 ml each was put into two filter tubes and centrifuged at 2000 rpm for 1 minute, and the blood was divided into the prepared filter tubes containing the ficoll. The mixture was centrifuged at 2500 rpm for 25 minutes. Here, only the peripheral blood mononuclear cell (PBMC) layer was separated, collected in a new 50 ml tube, adjusted to a volume of 50 ml with PBS, and centrifuged at 2000 rpm for 10 minutes. When there were many RBCs (red blood cells) in a pellet, an RBC lysis buffer was used.

After centrifugation, the supernatant was removed, and the pellet was released with 10 ml of RPMI (culture medium) (Hyclone). Thereafter, an appropriate amount of suspension was taken and used for cell counting, and after adjusting to a volume of 50 ml with RPMI culture medium, the remaining suspension was centrifuged at 2000 rpm for 10 minutes.

At this time, the RBC lysis buffer was as described below.

The RBC lysis buffer of 1 ml was added per 1×10⁸ to 2×10⁸ cells and tapped for 1 minute. After adding 15 to 20 ml of RPMI (culture medium) thereto, centrifugation was performed at 1500 rpm (250 to 500×G) for 7 minutes. When RBC removal was required further, the process described above was repeated 2 to 3 times.

### <Example 2> Sample peripheral blood mononuclear cell (PBMC) immune cell distribution

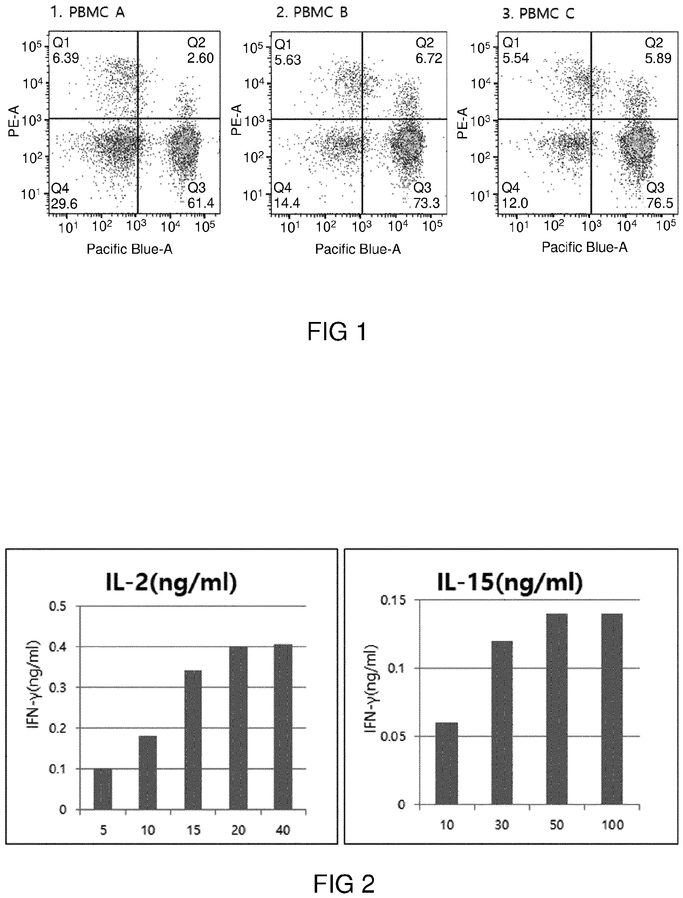

As described in Example 1 above, 40-60 cc of peripheral blood was divided into two filter tubes containing ficoll and then centrifuged at 2500 rpm for 25 minutes. Only the peripheral blood mononuclear cell (PBMC) layer was separated from the layered peripheral blood, collected in a new 50 ml tube, and washed with PBS. Cells obtained by discarding the supernatant were subject to a reaction with antibodies (CD56-PE, CD3-BV421) containing a fluorescent material and then analyzed by flow cytometry. Table 1 and FIG. 1 below show the results of immune cell distribution according to the antibodies.

**[Table 1]**

| | T (CD3+CD56-) | NK (CD3-CD56+) | NKT (CD3+CD56+) |
|---|---|---|---|
| 1 Sample A | 61.4 | 6.39 | 2.6 |
| 2 Sample B | 73.3 | 5.63 | 6.72 |
| 3 Sample C | 76.5 | 5.54 | 5.89 |

As shown in Table 1 and FIG. 1 above, it was confirmed that the overall distribution of the PBMC immune cells isolated from blood was NK cells 5% to 6%, NKT cells 2% to 7%, and T cells 60% to 80%.

### <Example 3> NK activity measurement test (IFN-γ measurement) for setting IL-2 and IL-15 cytokine concentrations

To confirm the effect and set the concentration of IL-2 and IL-15 cytokines as a preliminary experiment, monocytes obtained from peripheral blood according to Example 1 above were treated with respective concentrations of IL-2 and IL-15 cytokines in a flask coated with anti-CD16 antibody and in a medium containing RPMI 18 ml, plasma 2 ml, anti-CD56 5 ng/ml, anti-NKp46 5 ng/ml, and anti-NKp30 5 ng/ml and cultured for 7 days, and then all the cells were harvested. The collected cells were centrifuged at 2000 rpm for 5 minutes, and 1 ml of the supernatant was transferred to a new tube to measure cell activity. The cell activity was confirmed by measuring the amount of IFN-γ secretion by using an IFN-γ ELISA kit.

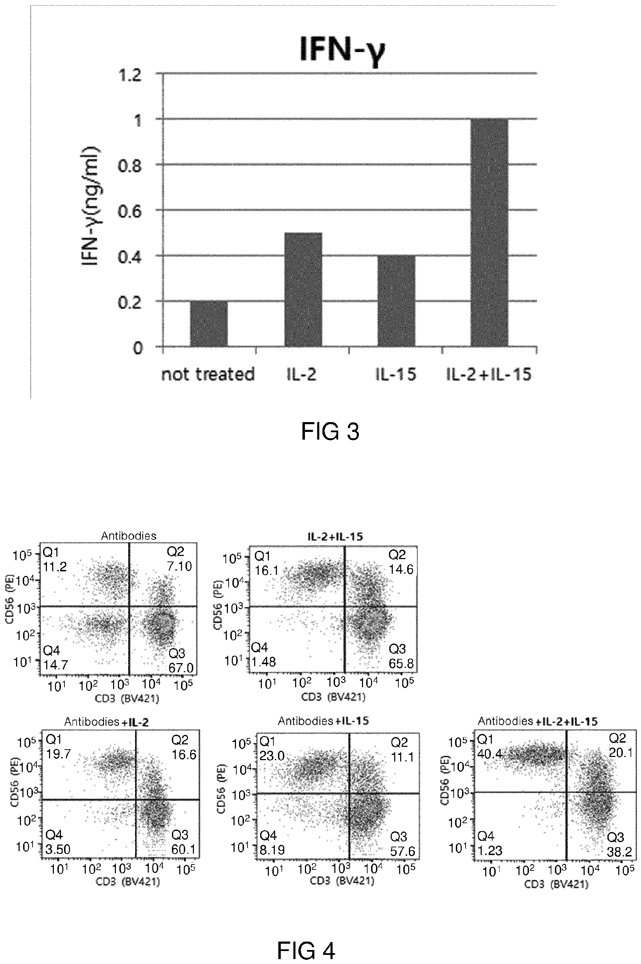

FIG. 2 shows the amount of IFN-γ secretion according to the concentration of IL-2 and IL-15 treatment in NK cells. FIG. 3 shows the activity according to the amount of secretion of IFN-γ after the treatment at the optimal IL-2 and IL-15 concentrations set by the results shown in FIG. 2.

Specifically, FIG. 2 shows that the amount of secretion of IFN-γ gradually increased when the IL-2 treatment concentration was 5, 10, 15, 20, or 40 (ng/ml), and the highest secretion was reached at 20 ng/ml. The secretion did not increase any further at 40 ng/ml. On the other hand, when the IL-15 concentrations of 10, 30, 50, and 100 (ng/ml) were compared, the secretion of IFN-γ showed the highest secretion at 50 ng/ml and did not increase any further at 100 ng/ml. The results from the two experiments confirmed that 20 ng/ml of IL-2 and 50 ng/ml of IL-15 are the most effective concentration in terms of cell activity.

FIG. 3 shows that the amount of IFN-γ secretion in the cultured cells treated with IL-2 + IL-15 was significantly higher than the amount of IFN-γ secretion in the cells treated with each of IL-2 and IL-15, confirming that the cell activity was increased.

### <Example 4> Measurement of immune cell distribution, cell viability, and cell count according to IL-2 and IL-15 treatment

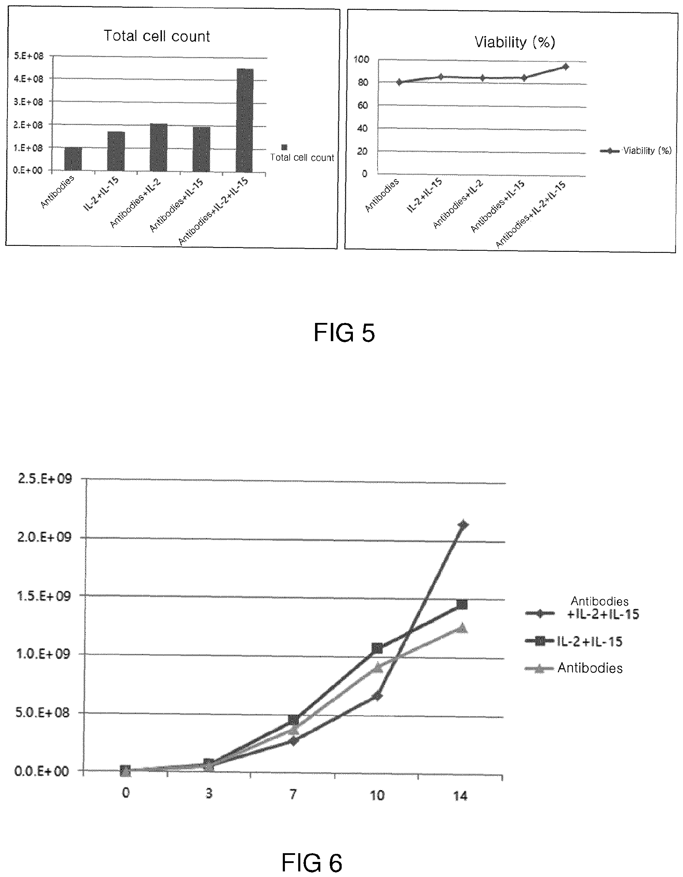

As a preliminary experiment for NK cell mass culture, to see the effect of each condition of antibody and IL-2 and IL-15 on NK cell ratio and cell viability or cell count, monocytes obtained from peripheral blood according to Example 1 above were treated under different conditions in a flask coated with anti-CD16 antibody and in a medium containing RPMI 18 ml and plasma 2 ml and then cultured for 7 days, and then all the cells were harvested to analyze the surface antigens of the NK cells . The treatment was performed for each of the group basically containing RPMI 18 ml and plasma 2 ml in a flask coated with anti-CD16 antibody to which only antibodies (anti-CD56 5 ng/ml, anti-NKp46 5 ng/ml, anti-NKp30 5 ng/ml) were added; the group basically containing RPMI 18 ml and plasma 2 ml in a flask coated with anti-CD16 antibody to which only IL-2 (20 ng/ml)+IL-15 (50 ng/ml) cytokines were added; the group basically containing RPMI 18 ml and plasma 2 ml in a flask coated with anti-CD16 antibody to which only antibodies (anti-CD56 5 ng/ml, anti-NKp46 5 ng/ml, anti-NKp30 5 ng/ml)+IL-2 (20 ng/ml) were added; the group basically containing RPMI 18 ml and plasma 2 ml in a flask coated with anti-CD16 antibody to which only antibodies (anti-CD56 5 ng/ml, anti-NKp46 5 ng/ml, anti-NKp30 5 ng/ml)+IL-15 (50 ng/ml) were added; and the group basically containing RPMI 18 ml and plasma 2 ml in a flask coated with anti-CD16 antibody to which antibodies (anti-CD56 5 ng/ml, anti-NKp46 5 ng/ml, anti-NKp30 5 ng/ml)+IL-2 (20 ng/ml)+IL-15 (50 ng/ml) were added, and the culture was performed for 7 days. Then, the culture was centrifuged at 2000 rpm for 10 minutes, and the cells obtained by removing the supernatant were subjected to a reaction with antibodies (CD56-PE, CD3-BV421) containing a fluorescent substance and analyzed by flow cytometry. The cell count was measured by using a hemocytometer after suspending the cells obtained by centrifugation in PBS and taking 10 µl of the cell suspension and mixing the same with 10 µl of Trypan Blue. Table 2 and FIG. 4 below show the results of the immune cell distribution according to the antibodies, and FIG. 5 shows the total cell count and cell viability.

**[Table 2]**

| | T (CD3+CD56-) | NK (CD3-CD56+) | NKT (CD3+CD56+) |
|---|---|---|---|
| Antibodies | 67.0 | 11.2 | 7.10 |
| IL-2+IL-15 | 65.8 | 18.1 | 14.6 |
| Antibodies+IL-2 | 60.1 | 19.7 | 16.6 |
| Antibodies+IL-15 | 57.6 | 23.0 | 11.1 |
| Antibodies+IL-2+IL-15 | 38.2 | 40.4 | 20.1 |

As shown in Table 2 and FIG. 4, the ratio of NK cells was higher in the mixture treated with IL-2 + IL-15 cytokine than in the sample treated with only the antibodies. However, it was confirmed that treating with the mixture prepared by combining antibodies + IL-2 + IL-15 was effective in increasing NK and NKT cells. In addition, as shown in FIG. 5, it was confirmed that the antibodies + IL-2 + IL-15 mixture showed a proliferation effect 2 to 4 times higher than other control groups, and the viability was also maintained high.

### <Example 5> NK immune cell mass culture method at a set concentration

### <5-1> Anti-CD16 coating

Anti-CD16 was coated by mixing 10 ml of PBS and 5 ng/ml of anti-CD16 in a T75 flask, leaving the resulting mixture at room temperature for 6 hours, and then storing the same in a refrigerator overnight (24 h). The coated flask should be used within one week and was used after removing PBS and washing once with PBS before use. Since the flask is coated, care should be taken not to dry the bottom. Pipetting was carefully performed not to pipette directly to the bottom of the flask.

### <5-2> Immune cell culture

First, in addition to the anti-CD16 coated T75 flask prepared in Example 5-1 above, RPMI medium, plasma, IL-2, IL-15, anti-CD56, anti-NKp46, and anti-NKp30 were prepared.

The cells were cultured in a T25 flask coated with anti-CD16 antibody when the number of cells in the cell suspension was 3×10⁷ or less, and in a T75 flask coated with anti-CD16 antibody when the number was 3×10⁷ or more. Specifically, RPMI 8ml, plasma 2 ml, IL-2 20 ng/ml, IL-15 50 ng/ml, anti-CD56 5 ng/ml, anti-NKp46 5 ng/ml, and anti-NKp30 5 ng/ml were added to a coated T75 flask, and cells were obtained by the method of Example 1 by removing the supernatant. After pipetting with 10 ml of RPMI, the cells were added to the flask and cultured in an incubator at 37°C and 5% CO₂ for 3 to 4 days according to the conditions of the cells.

### <5-3> Subculture

For subculture, a T175 or T225 flask, RPMI medium, albumin, IL-2, IL-15, anti-CD56, anti-NKp46, and anti-NKp30 were prepared.

### <5-3-1> Primary subculture (subculture from T75 flask to T175 flask)

The immune cells cultured in the T75 flask of Example 5-2 above were subcultured into a T175 flask. Specifically, the T75 flask was scraped with a scraper to detach cells attached to the bottom, and the clumped cells were released by pipetting. The prepared cell suspension 20 ml was added to a new T175 flask with RPMI 72 ml, albumin 8 ml, IL-2 20 ng/ml, IL-15 50 ng/ml, anti-NKp30 5 ng/ml, anti-NKp46 5 ng/ml, and anti-CD56 5 ng/ml and mixed with the culture medium. Then, the cells were cultured in an incubator at 37°C and 5% CO₂ for 3 to 5 days according to the conditions of the cells.

### <5-3-2> Secondary subculture (subculture from T175 flask to two T225 flasks)

Subculture was performed from a T175 flask to two T225 flasks. Specifically, the T175 flask was scraped with a scraper to separate the cells attached to the bottom, and the clumped cells were released by pipetting. The prepared cell suspension of 100 ml was added to each of two new flasks, 50 ml each, with RPMI 180 ml, albumin 20 ml, IL-2 20 ng/ml, and IL-15 50 ng/ml, and mixed with the culture medium. Then, the cells were cultured in an incubator at 37°C and 5% CO₂ for 5 to 7 days according to the conditions of the cells. At this time, the media was added according to the cell conditions, and care was taken not to exceed the final volume of 400 ml for each T225 flask.

### <Example 6> Cell number measurement test after culturing NK cells for 14 days at a set concentration

It was investigated whether the composition set for mass proliferation could allow for mass proliferation of NK cells and increase the distribution even after 14 days of culture. Specifically, 5×10⁷ lymphocytes isolated by the method of Example 1 above were added to the culture medium of each sample, additionally treated with the set composition, which was RPMI + albumin + antibodies (anti-CD56 5 ng/ml, anti-NKp46 5 ng/ml, anti-NKp30 5 ng/ml) + IL-2 (20 ng / ml) + IL-15 (50 ng / ml), and then cultured for 14 days. In addition, the control groups were the group treated with only RPMI + albumin + IL-2 (20 ng/ml) + IL-15 (50 ng/ml) cytokines and the group treated with only RPMI + albumin + antibodies (anti-CD56 5 ng/ml, anti-NKp46 5 ng/ml, anti-NKp30 5 ng/ml). In addition, the cells were subcultured according to the number of cells and cultured for 14 days by adding a culture medium. For cell counting, the cultured cells were harvested on each day, 10 µl of the suspended cells was taken and mixed with 10 µl of Trypan Blue, and then measurement was performed with a hemocytometer. FIG. 6 shows the results. As shown in FIG. 6, when cultured for 14 days in the prepared culture medium, the cells were proliferated to more than 2×10⁹ cells.

### <Example 7> FACS analysis test according to the composition of NK cell mass culture at a set concentration

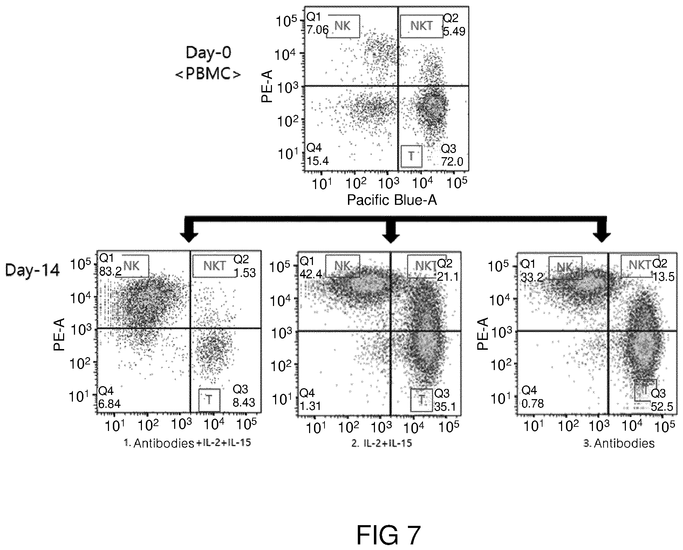

In the experiment (FACS) about the distribution of lymphocyte immune cells cultured according to Example 5-3-2, the cells were treated with RPMI + albumin + antibodies (anti-CD56 5 ng/ml, anti-NKp46 5 ng/ml, anti-NKp30 5 ng/ml) + IL-2 (20 ng/ml) + IL-15 (50 ng/ml) and cultured for 14 days, and the control groups were treated with only RPMI + albumin + IL-2 (20 ng/ml) + IL-15 (50 ng/ml) cytokines or with only RPMI + albumin + antibodies (anti-CD56 5 ng/ml, anti-NKp46 5 ng/ml, anti-NKp30 5 ng/ml), and each of the control groups was subcultured according to the number of cells for 14 days for comparison. The cells obtained by centrifugating at 2000 rpm for 10 minutes and removing the supernatant were subject to a reaction with antibodies (CD56-PE, CD3-BV421) containing a fluorescent material and analyzed by using flow cytometry. The results are shown in Table 3 and FIG. 7 below.

**[Table 3]**

| | T (CD3+CD56-) | NK (CD3-CD56+) | NKT (CD3+CD56+) |
|---|---|---|---|
| Antibodies+IL-2+IL15 | 8.43 | 83.2 | 1.53 |
| IL-2+IL15 | 35.1 | 42.4 | 21.1 |
| Antibodies | 52.5 | 33.2 | 13.5 |

As shown in Table 3 and FIG. 7 above, the results showed that the composition set for mass proliferation included 85% of NK and NKT cells and 8.5% of T cells even after 14 days of the mass culture, confirming that the NK cell ratio was significantly higher compared to the other control groups.

The present invention described above in detail with the preferred Examples of the present invention is not limited to the Examples above, and various modifications are possible by one of ordinary skill in the art within the scope of the technical principle of the present invention.

## Claims

1. A culture method of NK cell, the method comprising: 1) isolating a pellet of peripheral blood mononuclear cell (PBMC) containing immune cells from blood by using ficoll and centrifugation;
2) culturing the isolated PBMC in an anti-CD16 antibody-coated incubator in a medium containing RPMI culture medium, plasma, IL-2, IL-15, anti-CD56, anti-NKp46, and anti-NKp30;
3) subculturing the cultured PBMC in RPMI medium, albumin, IL-2, IL-15, anti-CD56, anti-NKp46, and anti-NKp30; and
4) treating the subcultured PBMC in a medium composition based on RPMI to which anti-NKp30, anti-NKp46 and anti-CD56 antibodies are added with a cytokine selected from a cytokine group consisting of IL-2, IL-15, and IL-2 + IL-15 to activate NK cells.

2. The culture method of NK cell according to claim 1, wherein the cytokine of the Step 4) is treated with IL-2 + IL-15.

3. The culture method of NK cell according to claim 2, wherein the cytokine of the Step 4) is treated with IL-2 20 ng/ml and IL-15 50 ng/ml.

4. The culture method of NK cell according to claim 1, wherein red blood cells are removed by treating an immune cell pellet with an RBC lysis buffer when isolating immune cells in the Step 1).

5. The culture method of NK cell according to claim 1, wherein the culturing in the Step 2) is carried out in a T25 flask coated with anti-CD16 antibody when the cell number is 3×10⁷ or less and in a T75 flask coated with anti-CD16 antibody when the cell number is 3×10⁷ or more by adding RPMI 8 ml, plasma 2 ml, IL-2 20 ng/ml, IL-15 50 ng/ml, anti-CD56 5 ng/ml, anti-NKp46 5 ng/ml, and anti-NKp30 5 ng/ml, suspending PBMC pellet in 10 ml RPMI and further adding the mixture, and culturing in an incubator at 37°C and 5% CO₂ for 3 to 4 days.

6. The culture method of NK cell according to claim 1, wherein the subculturing in the Step 3) is preferably carried out by adding the cell culture suspension of the Step 2) 20 ml, RPMI 72 ml, albumin 8 ml, IL-2 20 ng/ml, IL-15 50 ng/ml, anti-NKp30 5 ng/ml, anti-NKp46 5 ng/ml, and anti-CD56 5 ng/ml and culturing in an incubator at 37°C and 5% CO₂ for 3 to 5 days in primary subculture; and by adding primary subculture suspension 50 ml, RPMI 180 ml, albumin 20 ml, IL-2 20 ng/ml, IL-15 50 ng/ml, anti-NKp30 5 ng/ml, anti-NKp46 5 ng/ml, and anti-CD56 5 ng/ml and culturing in an incubator at 37°C and 5% CO₂ for 5 to 7 days in secondary subculture.
